# EUROPEAN PATENT SPECIFICATION

(11) **EP 1 882 478 B1**
(45) Date of publication and mention of the grant of the patent: **21.03.2012**
(21) Application number: 06253982.0
(22) Date of filing: 29.07.2006
(51) Int. Cl.: A61K 39/12, C07K 14/08, C07K 19/00

(54) **Fusion protein of porcine reproductive and respiratory syndrome virus as PRRS vaccine**
Fusionsproteine des reproduktiven und respiratorischen Syndrom Virus aus dem Schwein als PRRS Impfstoff
Protéine de fusion du virus du syndrome réspiratoire réproducteur porcin comme vaccin PRRS

(43) Date of publication of application: 30.01.2008
(73) Proprietor: Healthbanks Biotech Co., Ltd., Taipei City (TW)
(72) Inventor: Chang, Hsiu-Kang, Taipei City 106 (TW); Liao, Chao-Wei, Shin-Chu City 300 (TW)
(74) Representative: Tranter, Andrew David

(56) References cited:
- US-A1- 2004 247 617
- OSTROWSKI M ET AL: "Identification of neutralizing and nonneutralizing epitopes in the porcine reproductive and respiratory syndrome virus GP5 ectodomain" JOURNAL OF VIROLOGY, vol. 76, no. 9, May 2002 (2002-05), pages 4241-4250, XP002412599 ISSN: 0022-538X
- SNIJDER ERIC J ET AL: "Heterodimerization of the two major envelope proteins is essential for arterivirus infectivity." JOURNAL OF VIROLOGY, vol. 77, no. 1, January 2003 (2003-01), pages 97-104, XP002412600 ISSN: 0022-538X

## Description

### BACKGROUND OF THE INVENTION

### 1. Field of the Invention

The present invention relates to a fusion protein of PRRS subunit vaccine inducing PRRSV neutralization titers in pigs.

### 2. Description of Related Art

Porcine reproductive and respiratory syndrome is a porcine infectious disease that primarily strikes respiratory tracts of pigs at various ages and results in sows having reproductive dysfunction. PRRSV is a tough and resistant virus not prone to evoke antibodies having neutralization titers in the infectious pig. Besides, PRRSV is a RNA virus and reproduces easily on the basis of a simplified genetic system, so the probability of genetic mutations is very high. Furthermore, infections and pathogenesis pathway of PRRS virus can be sorted into two stages: (A) infections of epithelium tissues of upper and lower reproductive tracts; and (B) infections of monocytes and macrophages in tissues surrounding reproductive tracts. Thus, the host must have body fluid immunity as well as mucosal immunity with neutralization titers and also a cell-mediated immune response to facilitate removal of the infected viruses and strengthen the host protection mechanisms. However, it is not very easy for PRRSV infectious pig to have neutralization titers in natural conditions, hence typical antibodies basically have little effect on PRRSV, and they even induce mutations in viruses. Furthermore, in antibody-dependent enhancement of phagocytosis, the antibodies could only cause more severe PRRSV infections.

Taiwan Patent No.I-2289933 and United Stated States patent publication No. US 2004/0247617 disclose a target-cell-specific fusion protein, which utilizes a moiety and a functional domain of *Pseudomonas aeruginosa* exotoxin to fuse a PRRSV ORF7 nuclear protein fragment, with a KDEL signal peptide added on the carboxyl terminus. The fusion protein can be mass-produced in *E*. *coli.* When immunized the fusion protein to pigs, it is possible to decrease or to eliminate viremia after being PRRSV-challenged in the immunized pigs. The full text of the patent is incorporated herein.

In heterodimerization between ORF5 and ORF6 of PRRSV, epitope Cys-34 of ORF5 and epitope Cys-8 of ORF6 play a critical role in viral infection and the envelope assembly thereof. (Snijder Eric J., Jessica C. et al., Journal of Virology, January 2003, Vol. 77, No. 1:97-104). Besides, the consensus sequence of PRRSV ORF5 (YKNTHLDLIYNA) is an epitope between amino acid 38^{th} and amino acid 44^{th}, which is located at N-terminal extracellular domain of PRRSV ORF5 and had been identified as a neutralization epitope (Ostrowski M., J. A. Galeota, et al., Journal of Virology, May 2002, Vol. 76, No. 9:4241-4250).

Prior arts disclose constructing whole PRRSV ORF5 or ORF6 antigens between PE and KDEL. After immunization of these fusion proteins, the pigs suffered from severe inflammation in their lungs post being PRRSV-challenged, indicating that PRRSV ORF5 or ORF6 have an antigen-specific allergy effect. Manifestly, it is difficult to use them as PRRS vaccine antigens. Thus, to develop a vaccine and effectively protect pigs from PRRS infections, there are a lot of difficulties that have to be overcome. It should be designed for having a lower immunotoxicity and a higher neutralization titer.

U.S. Patent publication No. 2004/0247617 discloses a means for vaccination against PRRSV using a fusion protein comprising a) a Pseudomonas exotoxin A translocation domain, b) a peptide expressed from the PRRSV ORF7, and c) a carboxyterminal peptide sequence comprising KKDELRDELKDEL. Element a) is used in order to translocate the fusion protein into the cellular cytoplasm following cellular binding, and element c) is used in order to retain the protein in the endoplasmic reticulum. In immunization studies (example 2), two proteins were used, one having the KDEL type C terminus ('PE-DGDK') and one without ('PE-DGD'). Best protection against viral challenge was obtained with the PE-DGDK type fusion protein.

### SUMMARY OF THE INVENTION

In one aspect, the invention relates to a fusion protein of porcine reproductive and respiratory syndrome virus (PRRSV), comprising: a) a *Pseudomonas* exotoxin A (PE) peptide comprising a binding domain and a translocation domain, located at the N-terminus of the fusion protein; b) a fusion peptide of American strain PRRSV ORF6-2-26 (SEQ ID NO: 11) and ORF5-31-63 (SEQ ID NO: 10), or a fusion peptide of European strain PRRSV ORF6-M1-I28 (SEQ ID NO: 12) and ORF5-F31∼A64 (SEQ ID NO: 13); and c) a carboxyl terminal domain comprising the amino acid sequence of KDEL-KDEL-KDEL located at the C-terminus of the fusion protein, in which the capital letters 'KDEL' represent single letter amino acid codes.

In another aspect, the invention relates to a vaccine composition comprising an effective amount of a fusion protein as aforementioned and a pharmaceutically acceptable carrier.

In another aspect, the invention relates to a fusion protein as aforementioned for use in preventing PRRSV.

Further in another aspect, the invention relates to a vaccine composition as aforementioned for use in preventing PRRSV.

In the present invention, the nucleic acid sequence encoding the fusion protein containing a portion of PRRSV ORF5 and a portion of PRRSV ORF6 can be modified, and there is no particular limitation to the sequence, but it is preferably a nucleic acid sequence that can be expressed in large amounts in *E*. *coli* host-vector system, and the expressed proteins are identical to wild type ones. Taking the American strain of PRRSV as an example, preferably the modified nucleic acid sequence encoding the fusion protein is as seen in SEQ ID NO.1. For the European strains of PRRSV, preferably the modified Nucleic acid sequence encoding the fusion protein is SEQ ID NO:9.

A preferred embodiment of the portion of *Pseudomonas exotoxin* A binding and translocation domain of the fusion protein in the present invention is a detoxified *Pseudomonas exotoxin,* which is a fragment from *Pseudomonas exotoxin* A without domain III. Preferably, the fragment of *Pseudomonas exotoxin* A binding and translocation domain acts as a ligand moiety which is capable of reacting, recognizing or binding to a receptor on the target cell.

The pharmaceutical composition of the present invention can comprise a suitable adjuvant known in the art: dispersant, humectant (such as Tween 80), or sterile injections prepared with suspensions (such as sterile injection solutions or oily solutions). Sterile injection preparations can also be used in diluents or solvents of sterile injections or suspensions during innocuous injections, for example, in solutions of 1,3-butanediol. Acceptable carriers or solvents include mannitol, water, ringer solution, and isotonic sodium chloride solution. Besides, sterilized and fixed oils are used in prior arts as solvents or suspension media(for example, synthesized monoglycerides or diglycerides). Fatty acids (such as oleic acids or glyceride derivatives) and natural pharmaceutically acceptable oils (such as olive oil or castor oil, especially polyoxyethylated derivatives thereof) can be used in injectable preparations. The oil solutions or suspensions can also comprise long-chain alcohol diluents, dispersants, caboxylmethyl cellulose, or similar dispersants. Other commonly used surfactants like Tweens and Spans, or emulsifiers and bioavailability enhancers (usually used in manufacturing pharmaceutically acceptable alum solids, liquids or other dosage forms) can also be used for preparing purposes.

Compositions for oral administration can be any dosage form acceptable for oral administration, comprising, but not limited to, capsules, tablets, emulsions, water suspensions, dispersants, and solutions. In cases of tablets for oral administration purposes, the typical carriers include lactose and corn starch. A lubricant is often added, such as magnesium stearate. For oral administration with capsules, suitable diluents include lactose and corn starch. In cases of oral administration of water dispersants or emulsions, active ingredients could associate with emulsions or suspensions to suspend or disperse in the oil phase. Depending on needs, certain sweeteners, flavoring agents, or coloring agents can be added.

A nasal aerosol or inhalation composition can be prepared according to techniques well-known in the art of pharmaceutical formulation. For example, such a composition can be prepared as a solution in saline, employing benzyl alcohol or other suitable preservatives, absorption promoters to enhance bioavailability, fluorocarbons, and/or other solubilizing or dispersing agents known in the art. Compositions containing fusion proteins can also be administered in the form of suppositories for rectal administration.

The carriers of the pharmaceutical composition must be "acceptable", i.e. compatible to active ingredients in the formulation (preferably, capable of stabilizing the active ingredient) and not deleterious to the subject to be treated. Other examples of the carriers include colloidal silicon dioxide, magnesium stearate, cellose, sodium lauryl sulfate, and D&C yellow No. 10.

The pharmaceutical composition of the fusion protein in the present invention preferably comprises an immune adjuvant. The immune adjuvant used is not limited and can be any conventional one used in vaccines known in the art, comprising Alumigel and oil emulsion, such as Freund's FCA or FIA or mannide mono-oleate emulsifier (ISA720 or ISA206, SEPPIC^{®}, France), preferably ISA206.

Other objects, advantages, and novel features of the invention will become more apparent from the following detailed description when taken in conjunction with the accompanying drawings.

### BRIEF DESCRIPTION OF THE DRAWINGS

FIG 1 is a schematic illustration of PE-PQGAB fusion protein of Example 1;
FIG 2 is a flowchart of plasmid construction of PE(ΔIII)-PQGAB of Example 1;
FIG 3 is the electrophoresis diagram of the nucleic acid fragments synthesized according to Example 1, with four DNA fragments (a:70bp, b:129bp, c:186bp, d:204bp); and
FIG 4 is the plasmid map of PE(ΔIII)-PQGAB.
FIG 5 is the result of proteins induced in *E*. *coli* Host-vector system and extracted from inclusion bodies by 8M urea extraction. lane 0h, 2h: the total lysis samples at 0 hr and 2hr after IPTG induction of *E. coli* with pPE-PQGAB-K3; and lane 8M: 8M urea protein extraction of PE-PQGAB-K3.

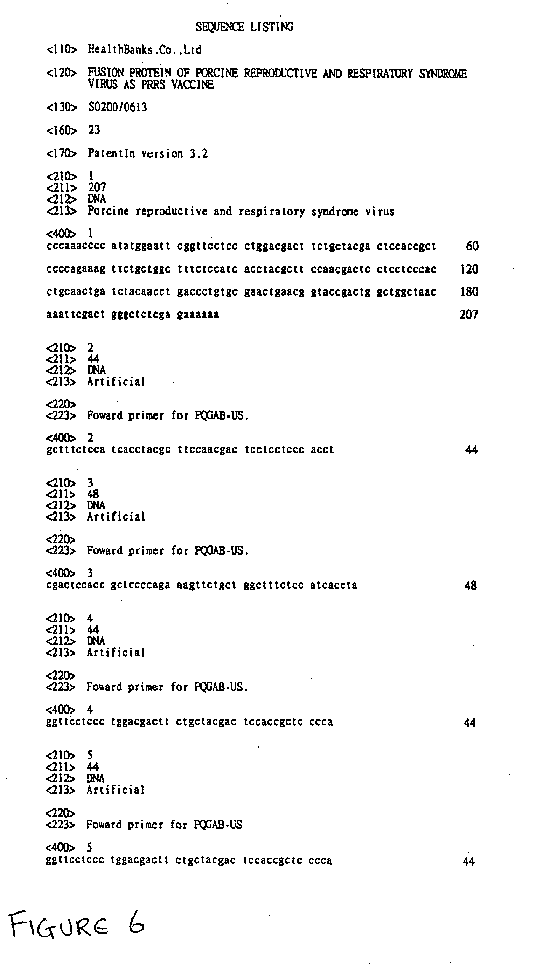

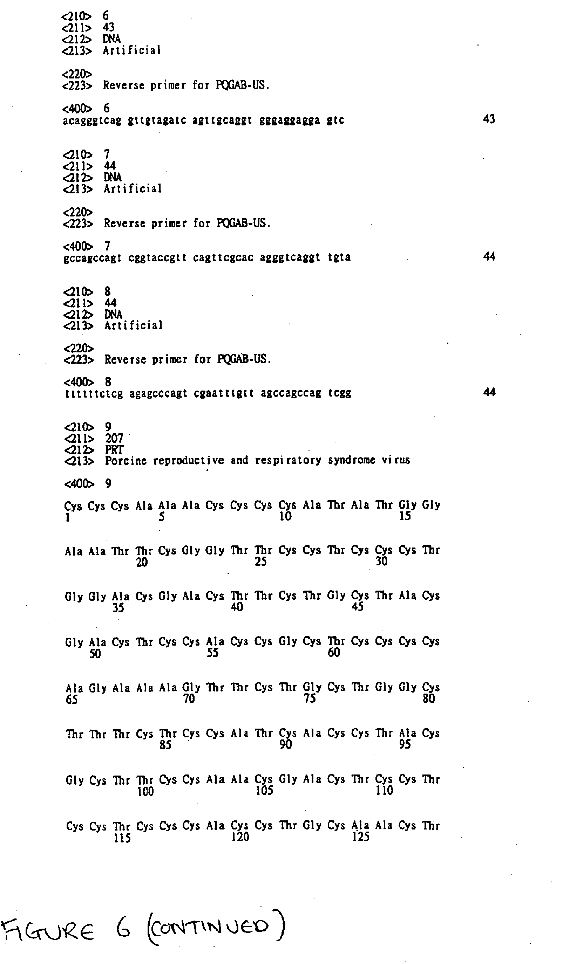

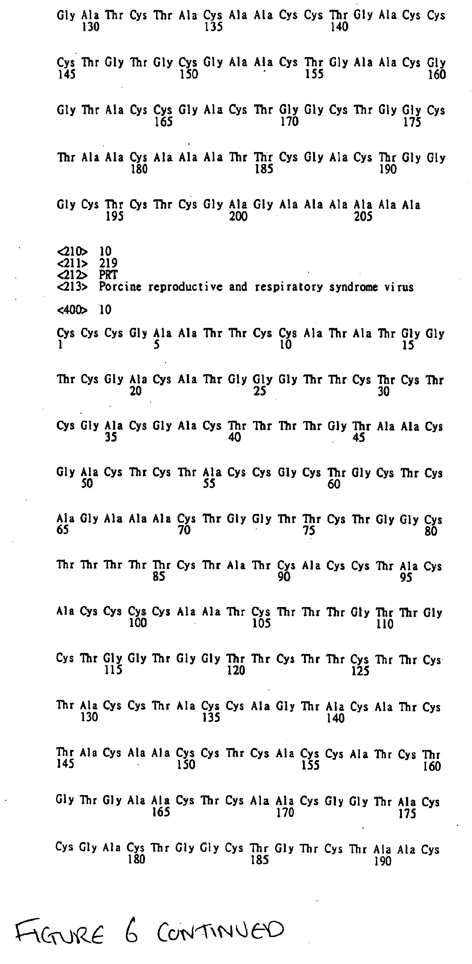

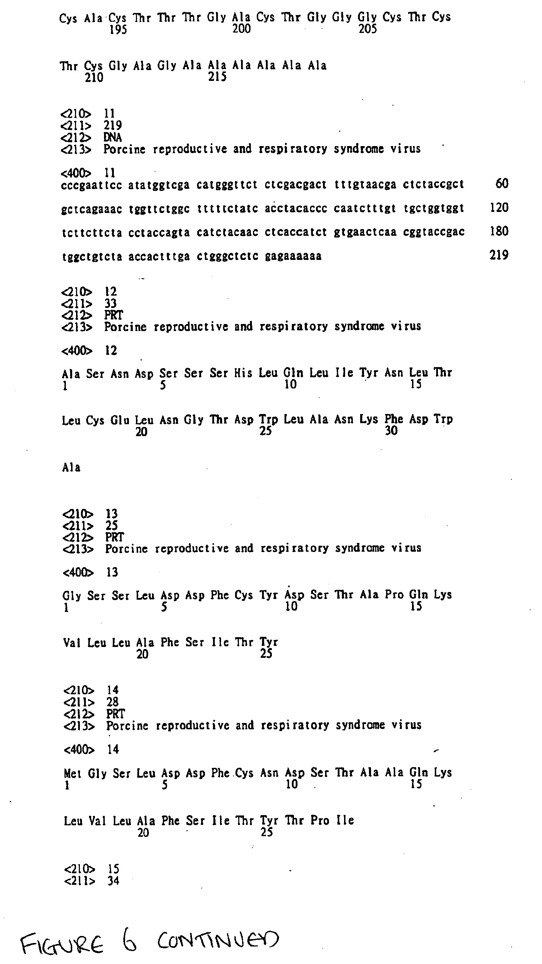

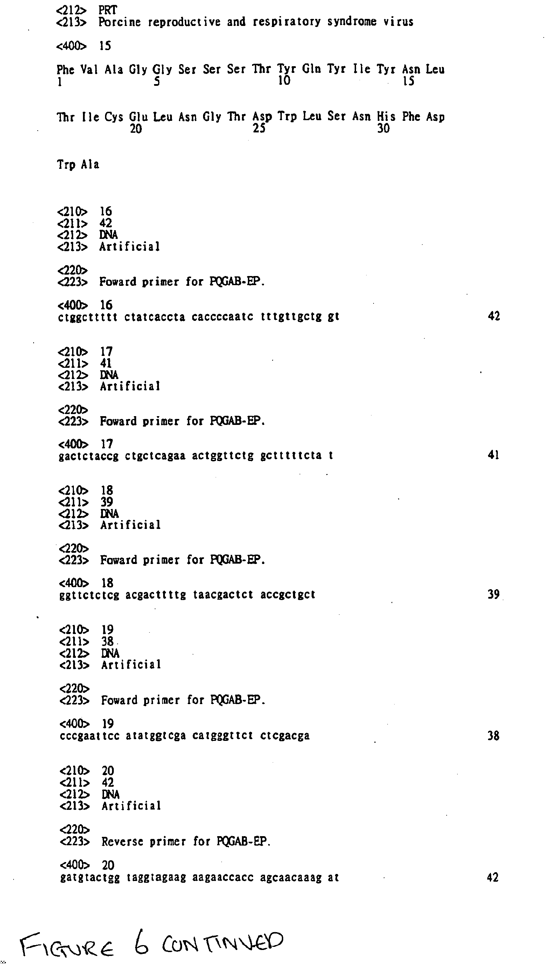

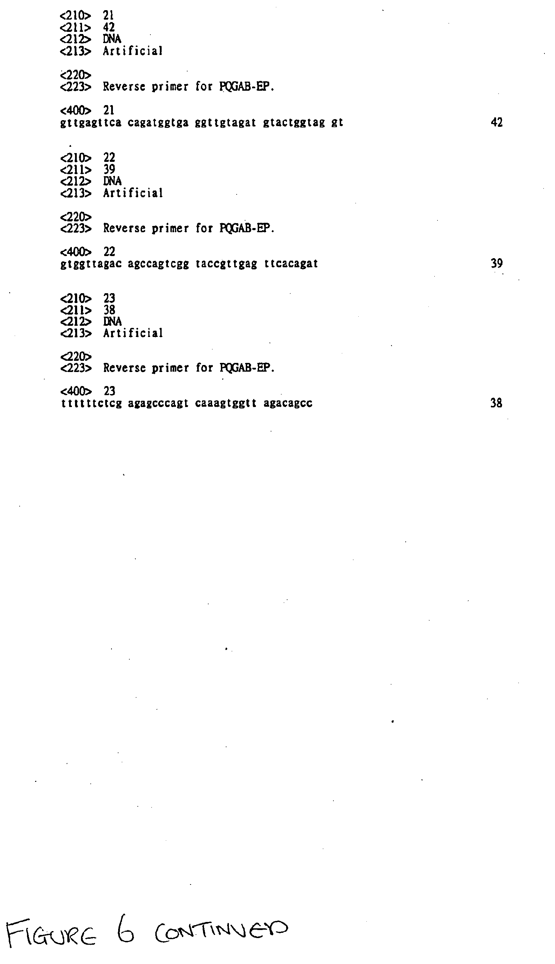

Fig. 6 shows the sequence of SEQ ID NO's 1 to 23.

### DETAILED DESCRIPTION OF THE PREFERRED EMBODIMENT

The feature of the present invention is based on a finding that, when most structure proteins in OR5 and ORF6 were removed, leaving dozens of N-terminal amino acids of ORF5 and ORF6, by which constructing a fusion peptide chain PQGAB, and then inserting the peptide chain between PE and K3 sequence was possible, it was confirmed that the fusion protein PE-PQGAB-K3 had serum neutralization titers by mice and porcine immunization tests.

The following examples are proposed to explain the present invention, but not set forth as to limit the scope thereof.

### Example 1: PQGAB fusion peptide of PRRSV American strain

The protein sequences of ORF5 and ORF6 of PRRSV were obtained from the National Center for Biotechnology Information (NCBI, USA) database. Based on the aforementioned mechanisms of viral infections, the regions of PRRSV that have neutralization titers are located at the N-terminus of ORF5 and ORF6. That is, amino acid residues 2 to 26 of ORF6 (SEQ ID NO.11), and amino acid residues 31 to 63 of ORF5 structural proteins (SEQ ID NO.10). The amino acid sequence resulting from the fusion of two peptides is illustrated below:
GSSLDDFCYDSTAPQKVLLAFSITYASNDSSSHLQLIYNLTLCE LNGTDWLANKFDWA

The sequence of PRRSV-ORF6-2 ∼ 26-ORF5-31 ∼ 63 fusion peptide was the combination of (ORF6)-G₂SSLDDFCYDSTAPQKVLLAFSITY₂₆ (SEQ ID NO.11) and (ORF5)-A₃₁SNDSSSHLQLIYNLTLCELNGTDWL ANKFDWA₆₃ (SEQ ID NO.10) peptides, wherein the fragment GSSLDDFC is designated "P", fragment YDSTAPQKVLLAFSITY "Q", fragment ASNDSSSHLQLIYNLTLC "A", and ELNGTDWLANKFDWA "B". Fragment PQ is a portion of ORF6, and Fragment AB is a portion of ORF5. G is the gap or bridge of PQ and AB ploypeptides. G can be the 27^{th} animo acid of ORF6 or any polypeptide fragment of ORF6 from 27^{th} to any linked codons. The position G can also be not added any amino acid within the polypeptides of PQ and AB.

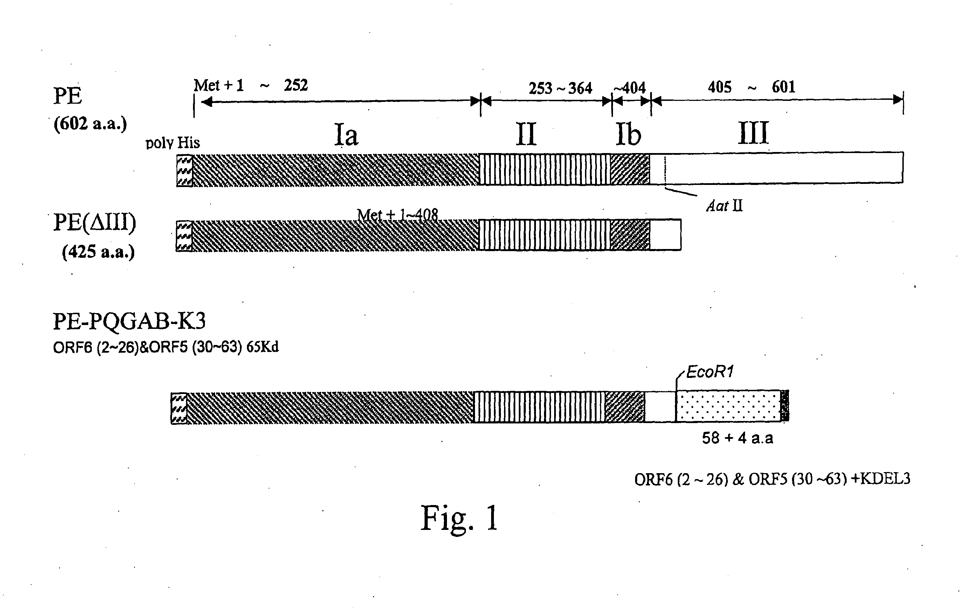

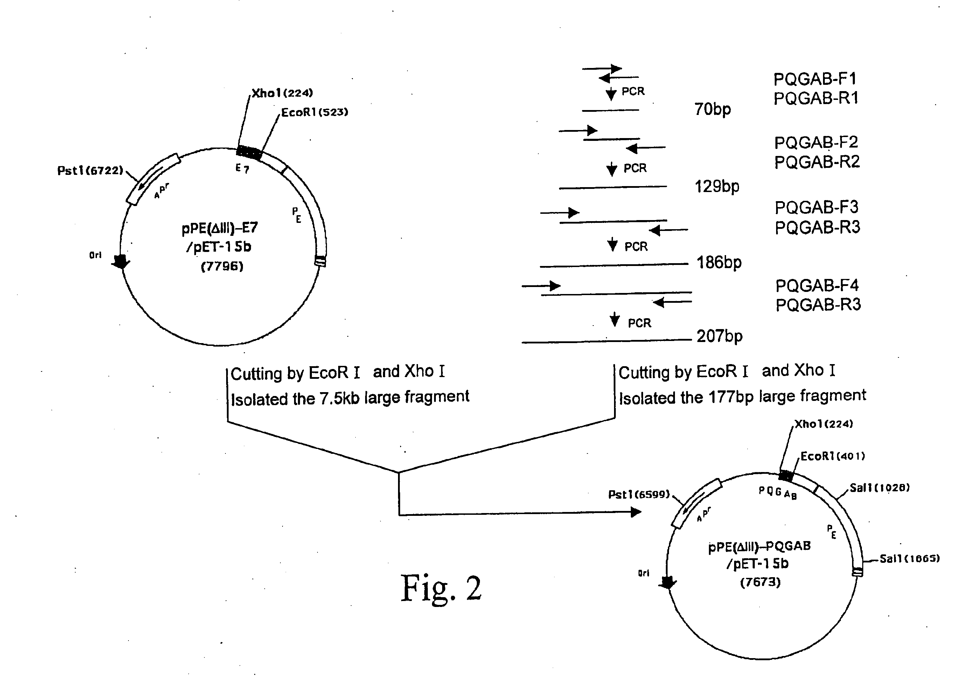

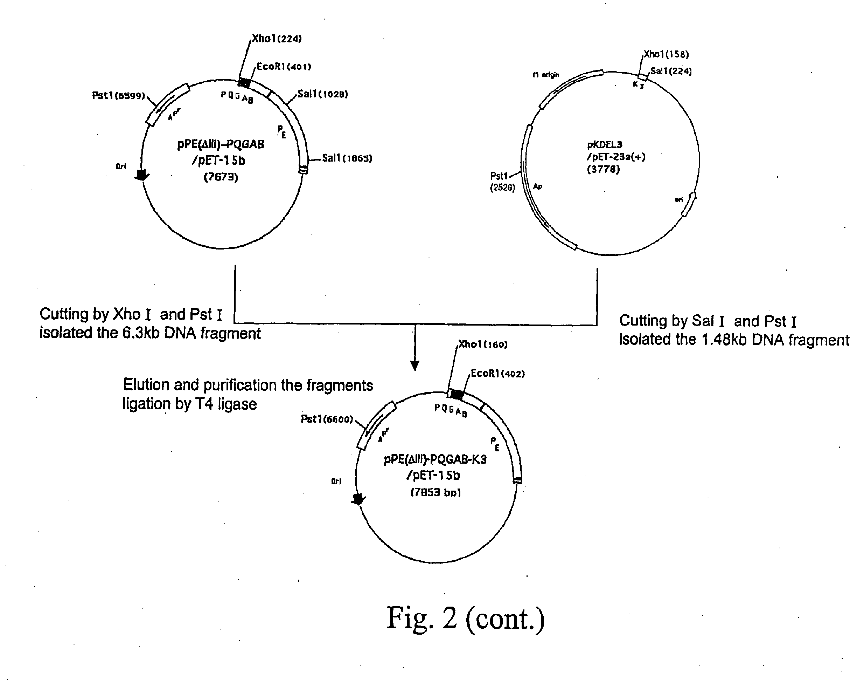

The example employs the PQGAB fusion peptide region to construct a key protein (epitope) capable of inducing neutralization titers and immune protection in order to obtain effects of inducing immune protection in vivo. The schematic illustration of PE-PQGAB-K3 fusion protein and the flowcharts of plasmids construction of PE(ΔIII)-PQGAB and PE(ΔIII)-PQGAB-K3 are shown in FIG. 1 and FIG. 2, respectively.

### Example 2

The preparation of nucleic sequence encoding PQGAB peptide is illustrated below. An amino acid corresponds to various sets of nucleotide triplets, so it is preferable to obtain corresponding nucleotide triplets from literature (such as http://www.kazusa.or.jp/codon) that is suitable to be expressed in the *E*. *coli* system instead of the corresponding nucleotide triplets not easy to be recognized and expressed by *E*. *coli.* Likewise, if the sequence encoding PQGAB peptide is to be expressed in yeast systems, the appropriate nucleotide triplets for expression in yeast systems (such as *Saccharomycesor Pichia spp*.) are preferred.

A corresponding sequence with nucleotide triplets suitable to be expressed in *E*. *coli* system was designated according to the amino acid sequence of PQGAB fusion protein. The 5' and 3' ends of the corresponding sequence were added by restriction sites for subsequent cloning. To improve efficiency of digestion and facilitate designing PCR primers, both ends of the sequence could be added to with nucleotide triplets with replicating bases, such as CCC, AAA, GGG, or TTT. The nucleic acid sequence encoding PQGAB fusion protein of the American strain of PRRSV is illustrated in SEQ ID NO.1.

There are totally 207 nucleotides in SEQ ID NO.1, and when it was cloned into a plasmid by restriction enzymes, some of the nucleotide triplets were cut off, leaving 180-186 nucleotides ligated to the plasmid.

When the target nucleic acid sequence encoding PQGAB fusion protein was identified, the restriction map of the nucleic acid sequence was analyzed by DNA Strider before synthesis, and then each end of the target sequence was linked to restriction site sequences for subsequent cloning, in accordance with the restriction map. The synthesized product of the target sequence must be digested by certain restriction enzymes before cloning, so it is preferable that any restriction site susceptible to the enzymes used be avoided in the structural region of the sequence. If the restriction sites subjected to cloning enzymes exist in the structural region of the target sequence, the target sequence must be re-designated such that different codons of the same amino acids were used, to eliminate restriction sites that were identical for cloning in the structural region of the target sequence.

Subsequently, the method disclosed in Taiwan Patent No.I-2289933 and United States patent publication No. US 2004/0247617 was used to modify the corresponding nucleotides codons of wild type amino acids sequence such that the wild type protein was mass expressed in by *E*. *coli* system. The 30 essence of modification is to modify wild type nucleic acid sequence such that the normally expressed amino acids were not affected, and expression in E. *coli* was kept effective. The modified nucleic acid sequence can be synthesized by PCR using a variety of primer pairs. The primers are numbered as shown in Table 1.

**Table 1: the corresponding numbers of primers for PQGAB antigens of PRRSV American Strain**

| **Target Antigen** | **Forward Primer** | **Seq. ID No.** | **Reverse primer** | **Seq. ID No.** |
|---|---|---|---|---|
| PQGAB-US | F1 | 2 | R1 | 6 |
| PQGAB-US | F2 | 3 | R2 | 7 |
| PQGAB-US | F3 | 4 | R3 | 8 |
| PQGAB-US | F4 | 5 | | |

The sequences of forward and reverse primers are shown as follows:
Forward primer F1 (SEQ ID NO: 2) corresponds to the nucleotides 79-122 of SEQ ID NO: 1, namely 5'-GCT TTC TCC ATC ACC TAC CGT TCC AAC GAC TCC TCC TCC CAC CT-3';
Forward primer F2 (SEQ ID NO: 3) corresponds to the nucleotides 48-95 of SEQ ID NO: 1, namely
   5'-C GAC TCC ACC GCT CCC CAG AAA GTT CTG CTG GCT TTC TCC ATC ACC TA-3';
Forward primer F3 (SEQ ID NO: 4) corresponds to the nucleotides 22-65 of SEQ ID NO: 1, namely
   5'-GGT TCC TCC CTG GAC GAC TTC TGC TAC GAC TCC ACC GCT CCC CA-3';
Forward primer F4(SEQ ID NO:5) corresponds to the nucleotides 1-40 of SEQ ID NO: 1, namely
   5'-CCC AAA CCC CAT ATG GAA TTC GGT TCC TCC CTG GAC GAC T-3' ;
Reverse primer R1(SEQ ID NO: 6) corresponds to the nucleotides 148-106 of SEQ ID NO: 1, namely
   5'-A CAG CGT CAG GTT GTA GAT CAG TTG CAG GTG GGA GGA GGA GTC-3';
Reverse primer R2(SEQ ID NO. 7) corresponds to the nucleotides 176-133 of SEQ ID NO: 1, namely
   5'-GC CAG CCA GTC GGT ACC GTT CAG TTC GCA CAG GGT CAG GTT GTA-3';
Reverse primer R3 (SEQ ID NO: 8) corresponds to the nucleotides 207-164 of SEQ ID NO:1, namely
   5'-TTT TTT CTC GAG AGC CCA GTC GAA TTT GTT AGC CAG CCA GTC GG-3';
wherein R1, R2 and R3 were reversely complementary sequences of a gene sequence.

The fragment synthesized with no DNA template was performed firstly. Forward primer F1 and reverse primer R1 were hybridized to each other, wherein 10-18 bases at 3' ends of each primer were designed complementary to each other, and the resultant complex was read and complemented by polymerase so as to obtain a double-stranded DNA template product.

After the first round of PCR, 0.01∼4 µl of the PCR product was taken as the template DNA of the second round of PCR, adding therein the second primer pair, i.e. forward primer F2 and reverse primer R2, 0.01∼4 µl each, in conjunction with needed dNTPs, reagents and Pfu polymerse, and the second round PCR was performed. Likewise, primer pair F3 and R3 were added therein and PCR was performed again; the procedures were repeated with primer pair F4 and R3, and thereby a modified PQGAB nucleic acid sequence having 207bp was obtained.

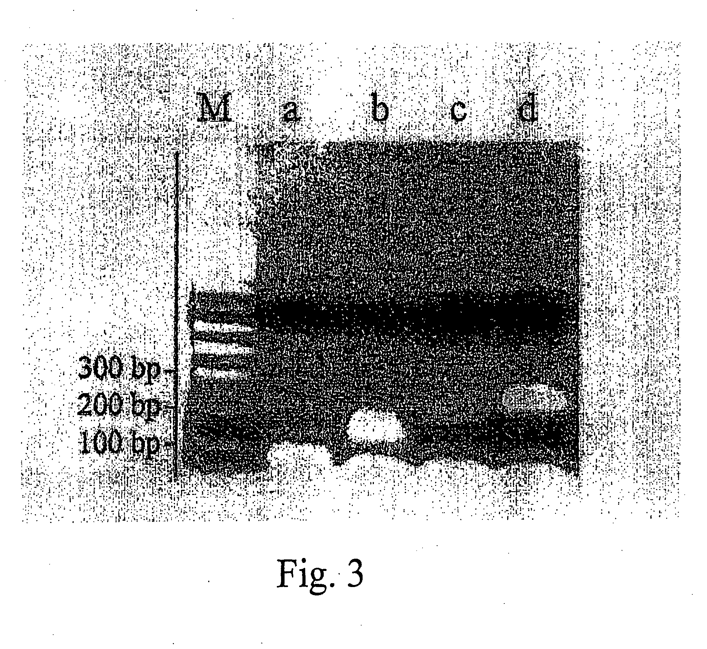

The synthesized nucleic acid fragments were subjected to electrophoresis and confirmed that they had the expected sizes. PQGAB-1(207bp), as shown in FIG 3; PQGAB generated 4 DNA fragments a, b, c, and d (a: 70bp b: 129bp c: 186bp, d: 207bp).

### Example 3: PQGAB fragment of PRRSV European strains

The design of the fusion protein in example 1 and 2 aimed at American strain PRRSV, but apart from American strain PRRSV, European strain and Australian strain is also very prevalent globally. Similarity of structural amino acids is not high, only 60-80%, so designs of other ORF5&ORF6 fusion proteins can be done in the same manner as example 1 and 2 to design and synthesize primers.

Taking PQGAB of PRRSV European strain as the example, the contained amino acid sequence of the fusion domain contained ORF6-M1 ∼ I28 + (SEQ ID NO. 12), and ORF5-F31∼A64 (SEQ ID NO.13) of the PRRSV European strain.

After the sequence was confirmed, preparation of PRRSV European strain fusion proteins was performed in the same manner as examples 1-2. The modified nucleic acid sequence was synthesized by PCR using a variety of primer pairs. The primers are numbered as shown in Table 2.

**Table 2: the corresponding numbers of primers for PQGAB antigens of PRRSV European Strain**

| **Target Antigen** | **Forward primer** | **Seq. ID No.** | **Reverse primer** | **Seq. ID No.** |
|---|---|---|---|---|
| PQGAB-EP | F1 | 14 | R1 | 18 |
| PQGAB-EP | F2 | 15 | R2 | 19 |
| PQGAB-EP | F3 | 16 | R3 | 20 |
| PQGAB-EP | F4 | 17 | R4 | 21 |

The target nucleic acid sequence encoding PQGAB-EP fusion protein were synthesized with those primers shown above in vitro, by following the procedure described in example 2. To improve efficiency of digestion and facilitate designing PCR primers, to both ends of the sequence were added nucleotide triplets with replicating bases, such as CCC, AAA, GGG, or TTT.

### Example 4: Construction of plasmids containing the target sequence

Taking the product from example 2 as an illustration. The synthesized 207-bp DNA fragment was digested with E*coR1 and Xho1,* linked to a *E.coli* plasmid containing a peptide sequence having functions of binding and translocation, and a carboxyl terminus peptide, and the resultant plasmid was pPE-PQGAB-K3.

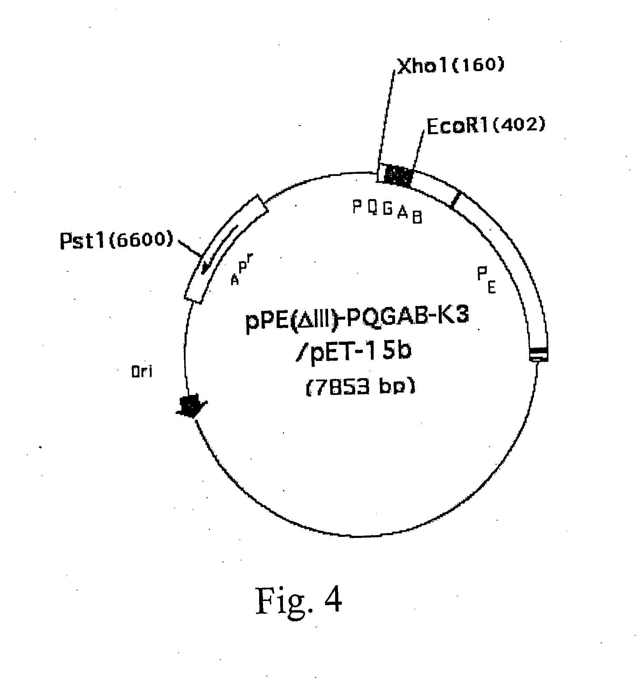

The pET15 plasmid having a T7 promoter and an antibiotic resistance(ampicilin) marker constructed therein can express the fusion protein of PRRSV PQGAB fragment and detoxified *Pseudomonas exotoxin* (*Pseudomonas exotoxin* A without domain III). The vector map is shown in FIG 4.

Finally, the above-mentioned plasmid was transformed into bacterial strains or cells capable of expressing the fusion proteins.

### Example 5: Expression and analysis of the target protein

Bacterial strains confirmed having the above mentioned plasmid contained both the plasmid and PQGAB gene in 90% of the population. The strains were prepared as glycerol stocks in 2-ml aliquots and stored at -70 °C. In a sterile environment, 2 ml of the stored stocks was inoculated in an autoclaved 500 ml flask containing 200 ml LB (+500 µg/ml Amp), shaken in a rotary incubator at 37°C,150 rpm for 10∼12 hours, and a culture was obtained. The liquid was cultured until OD600 reached 1.0±0.4.

In a sterile environment, 50 ml culture liquid was inoculated in each of eight 3000-ml flasks containing 1250 ml LB (+500 µg/ml Amp + 50 ml 10% Glucose), shaken at 37°C, 150 rpm for 2∼3 hours until OD 600 reached 0.3±0.1, 50 ppm IPTG was added, the culture was shaken again at 37°C, 150 rpm for 2 hours such that protein production was accomplished.

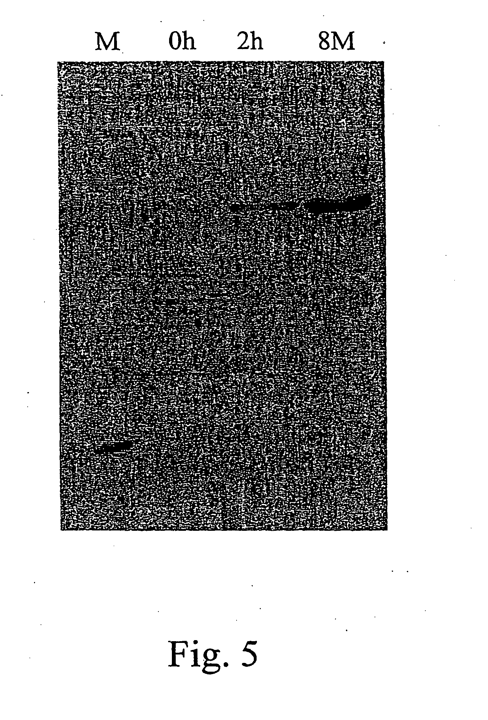

Then PE-PQGAB-K3 contained in inclusion bodies was dissolved by 8M urea extraction method, the extracted PE-PQGAB-K3 proteins are shown in FIG. 5. 300∼400mg antigen could be obtained from a 10-liter lot of the culture liquid. Obtained antigen was analyzed with Western blot, coomasie blue staining and SDS-PAGE electrophoresis, the density of the bands was measured by densitometer to quantify proteins contained in antigen solutions. 0.2±0.02 mg of the proteins were used as the main ingredient of a low-dosage injection in order to proceed immunization and virus-challenging.

### Example 6: Immunization and virus-challenging in pigs

In an SPF farm, pigs were grouped randomly into 3 groups, each having five pigs. Each group was bred in an isolation room equipped with air conditioning and circulation instruments. For pigs of PE-PQGAB-K3 immunization group aged 14 to 28 days, 2ml vaccine containing 1ml PE-PQGAB-K3 (containing 200µg proteins/injection) and emulsified in 1 ml ISA206 (SEPPIC^{®}, France) was injected intramuscularly, and the procedure of immunization was performed twice. The immunization group GP5&M was immunized with PE-ORF5-K3 PE-ORF6-K3(containing 200µg proteins/injection), respectively. The control group was bred without immunization.

Two weeks after the last inoculation, 100 mg ketamine solution was administered intramuscularly to tranquilize the pigs, then 1 ml 2% Lidocaine was dropped in the nasal cavities of the pigs to inhibit coughing reflex actions, and then the virus was inoculated in pigs nasally. Five pigs of each group were inoculated with 1 ml MD-1 strain PRRSV cultures having a 1×10⁷ TCID₅₀/ml dosage.

14 days after inoculation, the pigs were sacrificed to proceed with complete autopsy. Lung or liver samples were collected (from both parts of the head lobe central part and auxiliary part of caudate lobe) and fixed by 10% neutral buffered formaldehyde for subsequent tissue pathology examination. The examination was conducted in a blind fashion and evaluated on the basis of interstitial pneumonitis severity (Opriessnig T, P.G. Halbur, et al.; Journal of Virology, 76(2002):11837-11844, and Halbur, P.G., P.S. Paul, et al., 1996. J. Vet. Diagn. Investig. 8:11-20) in a scale from 0 to 6, wherein the severity increases with the number.

### Experimental results

Two weeks post second immunization, leukocytes from porcine blood were tested for PRRSV. The results indicated that viremia did not occur in any pig before PRRSV inoculating. The leukocyte samples were tested with RT-PCR at 3, 7, and 14 days post virus inoculating, respectively, and the results are shown in Table 3.

**Table 3: PRRSV viremia occurrence in pigs post PRRSV inoculating**

| Day | Control | PE-PQGAB-K3 | PE-ORF6-K3 |
|---|---|---|---|
| 3 | 3/5 | 3/5 | 2/5 |
| 7 | 3/4(death1*) | 2/5 | 2/4(death2*) |
| 14 | 3/4(death1*) | 2/5 | 2/4(death2*) |

| | | | |
|---|---|---|---|
| *identified with PRRSV viremia by RT-PCR before death All pigs, including those that had been sacrificed and the surviving after the two-week study, were dissected. Macroscopic examinations indicated that the lungs from virus-inoculated pigs of ORF5&ORF6 vaccine group and the control group showed more extensive lesions and severe interstitial pneumonitis, whereas the PE-PQGAB-K3 vaccine group of the present invention did not show as extensive lesions and severe interstitial pneumonitis. As shown in Table 4, the PE-PQGAB-K3 vaccine group of the present invention showed less severity in terms of interstitial pneumonitis than the control group and ORF5&ORF6 vaccine group. | | | |

**Table 4: comparisons of macroscopic lung lesions induced by PRRSV, 14 days post PRRSV inoculating**

| Pig No. | Control group Lesion index | PE-PQGAB-K3 vaccine group Lesion index | PE-ORF5-K3, PE-ORF6-K3 vaccine group Lesion index |
|---|---|---|---|
| 1 | 6* | 5 | 6 |
| 2 | 6 | 3 | 5 |
| 3 | 6 | 4 | 6 |
| 4 | 6 | 4 | 6 |
| 5 | 5 | 3 | 6 |
| Average | 5.75 ±0.50 | 3.80 ±0.84 | 5.80 ±0.45 |

| | | | |
|---|---|---|---|
| *: interstitial pneumonitis lesion index | | | |

**Table 5: macroscopic lung lesion indexes exhibited by the PE-PQGAB-K3 vaccine group are significantly lower than control group and ORF5&ORF6 vaccine group in view of biostatistics.**

| Group | Number of individuals | Total | Average | Variance |
|---|---|---|---|---|
| Control group | 5 | 29 | 5.8 | 0.2 |
| PE-PQGAB-K3 group | 5 | 19 | 3.8 | 0.7 |
| PE-ORF5&ORF6-K3 group | 5 | 29 | 5.8 | 0.2 |

| ANOVA | | | | | | |
|---|---|---|---|---|---|---|
| Variation source | SS | Degree of freedom | MS | F | P-value | Critical value |
| Inter-group | 13.33333 | 2 | 6.666667 | 18.18182 | 0.000233 | 3.88529 4 |
| Intra-group | 4.4 | 12 | 0.366667 | | | |
| total | 17.73333 | 14 | | | | |

The above experiments clearly indicate that PE-PQGAB-K3 of the present invention not only can effectively protect pigs from PRRSV infections, but also cause slighter interstitial pneumonitis than other vaccines (such as PE-ORF5-K3, PE-ORF6-K3).

The antibody titers variation in immunized pigs are shown in table 6. The A group has good IgG ELISA titers, but the IFA and NT titers are less than that of C group. Also, from table 5, it indicates that PRRSV ORF5 or ORF6 have an antigen-specific allergy effect after immunization and virus challenged. Manifestly, it is difficult to use them as PRRS vaccine antigens.

**Table 6: Serum titers**

| Group | coating antigen | | IFA titers | NT titers* |
|---|---|---|---|---|
| | PE(ΔIII) | PQGAB | | |
| | IgG-ELISA titers | (S/BK) | | |
| A PE-ABCF-K3 PE-PQGF-K3 | 12 | 80 | 8-16 | 8-16 |
| B Negative CTL | 1 | 1 | <8 | <8 |
| C PE-PQG1AB-K3 | 17 | 30 | 32-64 | 16-64 |

| | | | | |
|---|---|---|---|---|
| * :The neutralization titer is determined by the inhibition growth and proliferation of PRRSV under serial dilution sample added in MAC-10A cells culture system. | | | | |

## Claims

1. A fusion protein of porcine reproductive and respiratory syndrome virus (PRRSV), comprising:
a) a *Pseudomonas* exotoxin A (PE) peptide comprising a binding domain and a translocation domain, located at the N-terminus of the fusion protein;
b) a fusion peptide of American strain PRRSV ORF6-2∼26 (SEQ ID NO: 11) and ORF5-31-63 (SEQ ID NO: 10), or a fusion peptide of European strain PRRSV ORF6-M1-I28 (SEQ ID NO: 12) and ORFS-F31~A64 (SEQ ID NO: 13); and
c) a carboxyl terminal domain comprising the amino acid sequence of KDEL-KDEL-KDEL located at the C-terminus of the fusion protein, in which the capital letters 'KDEL' represent single letter amino acid codes.

2. The fusion protein of claim 1, wherein the fusion protein comprises an epitope capable of inducing neutralization titers.

3. A vaccine composition comprising an effective amount of a fusion protein according to claim 1 or 2 and a pharmaceutically acceptable carrier.

4. A fusion protein according to claim 1 or 2 for use in preventing porcine reproductive and respiratory syndrome virus (PRRSV).

5. A vaccine composition according to claim 3 for use in preventing porcine reproductive and respiratory syndrome virus (PRRSV).

6. The fusion protein of claim 1, comprising the fusion peptide of GSSLDDFCYDSTAPQKVLLAFSITYASNDSSSHLQLIYNLTLCELNGTDWLANKFDWA, in which the capital letters represent single letter amino acid codes.

7. The fusion protein of claim 1, comprising the fusion peptide of MGSLDDFCNDSTAAQKLVLAFSITYTPIFVAGGSSSTYQYIYNLTICELNGTDWLSNHFDWA, in which the capital letters represent single letter amino acid codes.

8. The fusion protein of claim 1, wherein the ORF5-31-63 is located between the ORF6-2∼26 and the carboxyl domain, and the ORF5-F31∼A64 is located between the ORF6-M1-I28 and the carboxyl domain.

## Patentansprüche

1. Fusionsprotein des reproduktiven und respiratorischen Syndrom Virus (PRRSV) des Schweins, aufweisend:
a) ein *Pseudomonas* Exotoxin A Peptid (PE), das eine Bindungsdomäne und eine TranslokationsDomäne aufweist, lokalisiert am N-Terminus des Fusionsproteins;
b) ein Fusionspeptid des amerikanischen Stammes PRRSV ORF6-2∼26 (SEQ ID Nr.: 11) und ORF5-31-63 (SEQ ID Nr.: 10) oder ein Fusionspeptid des europäische Stammes PRRSV ORF6-M1-I28 (SEQ ID Nr.: 12) und ORF5-F31∼A64 (SEQ ID Nr.: 13); und
c) eine carboxylterminale Domäne, die die Aminosäurensequenz KDEL-KDEL-KDEL aufweist, lokalisiert am C-Terminus des Fusionsproteins, wobei die ,KDEL' Einzelbuchstaben Aminosäure-Codes repräsentieren.

2. Fusionsprotein nach Anspruch 1, wobei das Fusionsprotein ein Epitop aufweist, das fähig ist, Neutralisationstiter zu erzeugen.

3. Impfstoffzusammensetzung, die eine wirksame Menge eines Fusionsproteins nach Anspruch 1 oder 2 und einen pharmazeutisch akzeptablen Trägerstoff aufweist.

4. Fusionsprotein nach Anspruch 1 oder 2 zur Verwendung bei der Unterdrückung von reproduktivem und respiratorischem Syndrom Virus des Schweins (PRRSV).

5. Impfstoffzusammensetzung nach Anspruch 3, zur Verwendung der Unterdrückung des reproduktiven und respiratorischen Syndrom Virus des Schweins (PRRSV).

6. Fusionsprotein nach Anspruch 1, aufweisend das Fusionspeptid GSSLDDFCYDSTAPQKVLLAFSITYASNDSSSHLQLIYNLTLCELNGTD WLANKFDWA, wobei die Großbuchstaben Einzelbuchstaben-Aminosäure-Codes repräsentieren.

7. Fusionsprotein nach Anspruch 1, aufweisend das Fusionspeptid MGSLDDFCNDSTAAQKLVLAFSITYTPIFVAGGSSSTYQYIYNLTICELN GTDWLSNHFDWA, wobei die Großbuchstaben Einzelbuchstaben-Aminosäure-Codes repräsentieren.

8. Fusionsprotein nach Anspruch 1, wobei das ORF5-31-63 zwischen dem TORF6-2-26 und der Carboxyl-Domäne Iokalisiert ist und wobei das ORF-5F31∼A64 zwischen dem ORF6-MM1-I28 und der Carboxyl-Domäne lokalisiert ist.

## Revendications

1. Protéine de fusion du virus du syndrome respiratoire et reproducteur porcin (VSRRP), comprenant :
a) un peptide de l'exotoxine A de *Pseudomonas* (EP) comprenant un domaine de liaison et un domaine de translocation, situés au niveau de l'extrémité N-terminale de la protéine de fusion ;
b) un peptide de fusion de l'ORF6-2∼26 (SEQ ID N° : 11) et l'ORF5-31-63 (SEQ ID N° : 10) du VSRRP de la souche américaine, ou un peptide de fusion de l'ORF6-M1-I28 (SEQ ID N° : 12) et l'ORF5-F31~A64 (SEQ ID N° : 13) du VSRRP de la souche européenne ; et
c) un domaine d'extrémité carboxyle-terminale comprenant la séquence d'acides aminés KDEL-KDEL-KDEL située au niveau de l'extrémité C-terminale de la protéine de fusion, dans lequel les majuscules 'KDEL' représentent des codes d'acides aminés à lettre unique.

2. Protéine de fusion selon la revendication 1, dans laquelle la protéine de fusion comprend un épitope capable d'induire des titres de neutralisation.

3. Composition de vaccin comprenant une quantité efficace d'une protéine de fusion selon la revendication 1 ou 2 et un vecteur pharmaceutiquement acceptable.

4. Protéine de fusion selon la revendication 1 ou 2 destinée à être utilisée dans la prévention du virus du syndrome respiratoire et reproducteur porcin (VSRRP).

5. Composition de vaccin selon la revendication 3 destinée à être utilisée dans la prévention du virus du syndrome respiratoire et reproducteur porcin (VSRRP).

6. Protéine de fusion selon la revendication 1, comprenant le peptide de fusion de la séquence GSSLDDFCYDSTAPQKVLLAFSITYASNDSSSHLQLIYNLTLCELNGTDWLAN KFDWA, dans laquelle les majuscules représentent des codes d'acides aminés à lettre unique.

7. Protéine de fusion selon la revendication 1, comprenant le peptide de fusion de la séquence MGSLDDFCNDSTAAQKLVLAFSITYTPIFVAGGSSSTYQYIYNLTICELNGTDW LSNHFDWA, dans laquelle les majuscules représentent des codes d'acides aminés à lettre unique.

8. Protéine de fusion selon la revendication 1, dans laquelle l'ORF5-31-63 est située entre l'ORF6-2∼26 et le domaine carboxyle, et l'ORF5-F31∼A64 est située entre l'ORF6-M1-I28 et le domaine carboxyle.


### SEQUENCE LISTING

<110> HealthBanks Biotech Co., Ltd
<120> FUSION PROTEIN OF PORCINE REPRODUCTIVE AND RESPIRATORY SYNDROME VIRUS AS PRRS VACCINE
<130> ADT35146P.EPA
<140> EP06253982.0
   <141> 2006-07-29
<160> 21
<170> PatentIn version 3.2
<210> 1
   <211> 207
   <212> DNA
   <213> Porcine reproductive and respiratory syndrome virus
<400> 1 
cccaaacccc atatggaatt cggttcctcc ctggacgact tctgctacga ctccaccgct 60
ccccagaaag ttctgctggc tttctccatc acctacgctt ccaacgactc ctcctcccac 120
ctgcaactga tctacaacct gaccctgtgc gaactgaacg gtaccgactg gctggctaac 180
aaattcgact gggctctcga gaaaaaa 207
<210> 2
   <211> 44
   <212> DNA
   <213> Artificial
<220>
   <223> Foward primer for PQGAB-US.
<400> 2
   gctttctcca tcacctacgc ttccaacgac tcctcctccc acct 44
<210> 3
   <211> 48
   <212> DNA
   <213> Artificial
<220>
   <223> Foward primer for PQGAB-US.
<400> 3
   cgactccacc gctccccaga aagttctgct ggctttctcc atcaccta 48
<210> 4
   <211> 44
   <212> DNA
   <213> Artificial
<220>
   <223> Foward primer for PQGAB-US.
<400> 4
   ggttcctccc tggacgactt ctgctacgac tccaccgctc ccca 44
<210> 5
   <211> 44
   <212> DNA
   <213> Artificial
<220>
   <223> Foward primer for PQGAB-US
<400> 5
   ggttcctccc tggacgactt ctgctacgac tccaccgctc ccca 44
<210> 6
   <211> 43
   <212> DNA
   <213> Artificial
<220>
   <223> Reverse primer for PQGAB-US.
<400> 6
   acagggtcag gttgtagatc agttgcaggt gggaggagga gtc 43
<210> 7
   <211> 44
   <212> DNA
   <213> Artificial
<220>
   <223> Reverse primer for PQGAB-US.
<400> 7
   gccagccagt cggtaccgtt cagttcgcac agggtcaggt tgta 44
<210> 8
   <211> 44
   <212> DNA
   <213> Artificial
<220>
   <223> Reverse primer for PQGAB-US.
<400> 8
   ttttttctcg agagcccagt cgaatttgtt agccagccag tcgg 44
<210> 9
   <211> 219
   <212> DNA
   <213> Porcine reproductive and respiratory syndrome virus
<400> 9 
cccgaattcc atatggtcga catgggttct ctcgacgact tttgtaacga ctctaccgct 60
gctcagaaac tggttctggc tttttctatc acctacaccc caatctttgt tgctggtggt 120
tcttcttcta cctaccagta catctacaac ctcaccatct gtgaactcaa cggtaccgac 180
tggctgtcta accactttga ctgggctctc gagaaaaaa 219
<210> 10
   <211> 33
   <212> PRT
   <213> Porcine reproductive and respiratory syndrome virus
<400> 10 
Ala Ser Asn Asp Ser Ser Ser His Leu Gln Leu Ile Tyr Asn Leu Thr
1               5                   10                  15

Leu Cys Glu Leu Asn Gly Thr Asp Trp Leu Ala Asn Lys Phe Asp Trp
            20                  25                  30

Ala
 <210> 11
   <211> 25
   <212> PRT
   <213> Porcine reproductive and respiratory syndrome virus
<400> 11 
Gly Ser Ser Leu Asp Asp Phe Cys Tyr Asp Ser Thr Ala Pro Gln Lys
1               5                   10                  15

Val Leu Leu Ala Phe Ser Ile Thr Tyr
            20                  25
<210> 12
   <211> 28
   <212> PRT
   <213> Porcine reproductive and respiratory syndrome virus
<400> 12 
Met Gly Ser Leu Asp Asp Phe Cys Asn Asp Ser Thr Ala Ala Gln Lys
1               5                   10                  15

Leu Val Leu Ala Phe Ser Ile Thr Tyr Thr Pro Ile
            20                  25
<210> 13
   <211> 34
   <212> PRT
   <213> Porcine reproductive and respiratory syndrome virus
<400> 13 
Phe Val Ala Gly Gly Ser Ser Ser Thr Tyr Gln Tyr Ile Tyr Asn Leu
1               5                   10                  15

Thr Ile Cys Glu Leu Asn Gly Thr Asp Trp Leu Ser Asn His Phe Asp
            20                  25                  30

Trp Ala
<210> 14
   <211> 42
   <212> DNA
   <213> Artificial
<220>
   <223> Foward primer for PQGAB-EP.
<400> 14
   ctggcttttt ctatcaccta caccccaatc tttgttgctg gt 42
<210> 15
   <211> 41
   <212> DNA
   <213> Artificial
<220>
   <223> Foward primer for PQGAB-EP.
<400> 15
   gactctaccg ctgctcagaa actggttctg gctttttcta t 41
<210> 16
   <211> 39
   <212> DNA
   <213> Artificial
<220>
   <223> Foward primer for PQGAB-EP.
<400> 16
   ggttctctcg acgacttttg taacgactct accgctgct 39
<210> 17
   <211> 38
   <212> DNA
   <213> Artificial
<220>
   <223> Foward primer for PQGAB-EP.
<400> 17
   cccgaattcc atatggtcga catgggttct ctcgacga 38
<210> 18
   <211> 42
   <212> DNA
   <213> Artificial
<220>
   <223> Reverse primer for PQGAB-EP.
<400> 18
   gatgtactgg taggtagaag aagaaccacc agcaacaaag at 42
<210> 19
   <211> 42
   <212> DNA
   <213> Artificial
<220>
   <223> Reverse primer for PQGAB-EP.
<400> 19
   gttgagttca cagatggtga ggttgtagat gtactggtag gt 42
<210> 20
   <211> 39
   <212> DNA
   <213> Artificial
<220>
   <223> Reverse primer for PQGAB-EP.
<400> 20
   gtggttagac agccagtcgg taccgttgag ttcacagat 39
<210> 21
   <211> 38
   <212> DNA
   <213> Artificial
<220>
   <223> Reverse primer for PQGAB-EP.
<400> 21
   ttttttctcg agagcccagt caaagtggtt agacagcc 38